# EUROPEAN PATENT APPLICATION

(11) **EP 4 488 361 A1**
(43) Date of publication of application: **08.01.2025**
(21) Application number: 23774458.6
(22) Date of filing: 03.03.2023
(51) Int. Cl.: C12N 5/00, C07K 14/78, C12N 11/00

(54) **BIOINK AND METHOD FOR PRODUCING STRUCTURE**

(30) Priority: 24.03.2022 JP 2022048329
(71) Applicant: Toppan Holdings Inc., Tokyo 110-0016 (JP); OSAKA UNIVERSITY, Suita-shi Osaka 565-0871 (JP)
(72) Inventor: KITANO Shiro, Tokyo 110-0016 (JP); MATSUSAKI Michiya, Suita-shi, Osaka 565-0871 (JP); XIE Zhengtian, Suita-shi, Osaka 565-0871 (JP)
(74) Representative: TBK
(86) International application number: PCT/JP2023/008116
(87) International publication number: WO 2023/181859

(57) **Abstract**

The present invention relates to a bioink containing a fragmented extracellular matrix component.

## Description

### Technical Field

The present invention relates to a bioink and a method for producing a structure.

### Background Art

In recent years, in fields such as regenerative medicine and an assay system for drugs which require an environment close to that of the living body, it has been demonstrated that the use of three-dimensional cellular tissues that are three-dimensionally organized has advantages over the use of cells grown on a plate. Therefore, various techniques for constructing three-dimensional cellular tissues in vitro have been developed.

A three-dimensional (3D) bioprinting technique has recently emerged, and attempts have been made to use 3D bioprinting to construct complex tissues and organs. A lot of 3D bioprinting techniques have been proposed so far (for example, Patent Literature 1 to 4).

### Citation List

### Patent Literature

[Patent Literature 1] United States Patent Application, Publication No. 2014/0120192
[Patent Literature 2] PCT International Publication No. WO2013/040078
[Patent Literature 3] Japanese Unexamined Patent Publication No. 2018-522587
[Patent Literature 4] Japanese Patent No. 6791960

### Summary of Invention

### Technical Problem

In tissue construction by 3D printing, the physical properties of the structure after formation are mainly attributed to the properties of the bioink used for printing. As a bioink, a composition containing, as a main component, an RGD peptide of fibrin, collagen, or the like, or a hydrogel such as alginic acid, is generally used. The structure formed from the bioink had room for improvement in terms of strength.

An object of the present invention is to provide a bioink that is capable of forming a structure having high strength.

### Solution to Problem

The present invention provides the following [1] to [8].
[1] A bioink comprising:
   a fragmented extracellular matrix component.
[2] The bioink according to [1], wherein the fragmented extracellular matrix component includes a fragmented collagen component.
[3] The bioink according to[1] or [2], further comprising:
   a structure forming material.
[4] The bioink according to [3], wherein the structure forming material includes an extracellular matrix component.
[5] The bioink according to [4], wherein the extracellular matrix component includes collagen.
[6] The bioink according to any one of [1] to [5], wherein a content of the fragmented extracellular matrix component is 5 ng/mL or more and 30 ng/mL or less based on a total amount of the bioink.
[7] The bioink according to any one of [1] to [6], wherein the fragmented extracellular matrix component has a particle size of less than 40 µm.
[8] A method for producing a structure, comprising:
   carrying out printing with the bioink according to any one of [1] to [7] and; and
   solidifying the bioink used for the printing.

### Advantageous Effects of Invention

According to the present invention, it is possible to provide a bioink that is capable of forming a structure having high strength.

### Brief Description of Drawings

FIG. 1 shows photomicrographic images that show results obtained by observing particle diameters of particles in a solution (GG bath) containing trisodium citrate (TSC) and gellan gum.
FIG. 2 is a graph showing a relationship between the average size (µm) of particles in the GG bath and the TSC concentration (mol/L).
FIG. 3 is a graph showing results obtained by measuring the tensile strength of a structure produced using a bioink.
FIG. 4 is a photographic image showing results obtained by microscopic observation of a fragmented collagen component (CMF) in phosphate buffered saline (PBS).
FIG. 5(A) is a photographic image showing results obtained by the microscopic observation of the fragmented collagen component (CMF) in a filtered bioink, and FIG. 5(B) is an enlarged photographic image of FIG. 5(B).
FIG. 6 is a graph showing results obtained by measuring the tensile strength of a structure produced using a bioink.
FIG. 7 is a graph showing results obtained by measuring the Young's modulus (Modulus) of a structure produced using a bioink.

### Description of Embodiments

Hereinafter, embodiments for executing the present invention will be described in detail. However, the present invention is not limited to the following embodiments.

### [Bioink]

A bioink according to the present embodiment contains a fragmented extracellular matrix component. The bioink is an ink composition that can form a structure by bioprinting. Specifically, the bioink is an ink composition that contains a biocompatible material, is liquid at the time of ejecting by a printer, and solidifies after being ejected from a printer, according to stimulation, elapse of time, and the like.

The "fragmented extracellular matrix component" in the present specification can be obtained by fragmenting an extracellular matrix component. The fragmented extracellular matrix component may be dispersed in the bioink. It is considered that in the bioink, the fragmented extracellular matrix component plays a role as a reinforcement filler. Fragmented extracellular matrix components intertwine with each other to form a filler network in the structure, which presumably contributes to improving the strength of the final structure. However, the mechanism for improving the strength of the structure is not limited to this.

In addition, since the bioink contains a fragmented extracellular matrix component, it is possible to increase the strength of the structure obtained by bioprinting while making it possible for the bioink to be smoothly ejected from a printer.

The extracellular matrix component is an aggregate of extracellular matrix molecules, which is formed from multiple extracellular matrix molecules. The extracellular matrix molecule may be a substance that is present outside cells in a multicellular organism. Any substance can be used as an extracellular matrix molecule as long as it does not adversely affect cell growth and the formation of cell aggregates. Specific examples of the extracellular matrix molecule include, but are not limited to, collagen, laminin, fibronectin, vitronectin, elastin, tenascin, entactin, fibrillin, and proteoglycan. As the extracellular matrix component, one kind of these extracellular matrix molecules may be used alone, or two or more kinds thereof may be used in combination.

The extracellular matrix molecule may be a modified molecule and a variant of the extracellular matrix molecules described above or may be a polypeptide such as a chemically synthesized peptide. The extracellular matrix molecule may have repeats of a sequence represented by Gly-X-Y, which is characteristic of collagen. Here, Gly represents a glycine residue, and X and Y each independently represent any amino acid residue. A plurality of Gly-X-Y sequences may be the same or different from each other. In a case where repetitions of the sequence represented by Gly-X-Y are provided, the restriction on the disposition of the molecular chain is reduced. In an extracellular matrix molecule having repeats of the sequence represented by Gly-X-Y, the proportion of the sequence represented by Gly-X-Y may be 80% or more in the total amino acid sequence, and it is preferably 95% or more. In addition, the extracellular matrix molecule may also be a polypeptide having an RGD sequence. The RGD sequence refers to a sequence represented by Arg-Gly-Asp (arginine residue - glycine residue - aspartic acid residue). Examples of the extracellular matrix molecule containing the sequence represented by Gly-X-Y and the RGD sequence include collagen, fibronectin, vitronectin, laminin, and cadherin.

Examples of the collagen include a fibrous collagen and a non-fibrous collagen. The fibrous collagen means collagen that is the main component of the collagen fiber, and specific examples thereof include type I collagen, type II collagen, and type III collagen. Examples of the non-fibrous collagen include type IV collagen.

Examples of the proteoglycan include, but are not limited to, chondroitin sulfate proteoglycan, heparan sulfate proteoglycan, keratan sulfate proteoglycan, and dermatan sulfate proteoglycan.

Due to the fact that the effect due to the present invention is more remarkable, the extracellular matrix component may include at least one selected from the group consisting of collagen, laminin, and fibronectin, and it is preferable to include collagen. The collagen is preferably a fibrous collagen and more preferably type I collagen. As the fibrous collagen, a commercially available collagen may be used, and specific examples thereof include a porcine skin-derived type I collagen, manufactured by NH Foods Ltd.

The extracellular matrix component may be an extracellular matrix component derived from an animal. Examples of the animal species from which the extracellular matrix component is derived include, but are not limited to, a human, a pig, and cattle. Regarding the extracellular matrix component, a component derived from one species of animal may be used, or components derived from a plurality of animal species may be used in combination.

In the present specification, the term "fragmentation" means reducing a size of an aggregate of an extracellular matrix molecule to a smaller size. Fragmentation may be carried out under conditions in which bonds within an extracellular matrix molecule are cleaved or may be carried out under conditions in which bonds within an extracellular matrix molecule are not cleaved. The fragmented extracellular matrix component may include a defibrated extracellular matrix component (a defibrated extracellular matrix component) which is a component obtained by defibrating the extracellular matrix component described above by the application of physical force. Defibration is one aspect of fragmentation and is carried out, for example, under conditions in which bonds within an extracellular matrix molecule are not cleaved.

A method of fragmenting an extracellular matrix component is not limited. Regarding a method of defibrating an extracellular matrix component, the extracellular matrix component may be defibrated by the application of physical force with, for example, an ultrasonic homogenizer, a stirring type homogenizer, or a high-pressure homogenizer. In a case where a stirring type homogenizer is used, the extracellular matrix component may be homogenized as it is or may be homogenized in an aqueous medium such as saline. In addition, it is also possible to obtain a millimeter-sized or nanometer-sized defibrated extracellular matrix component by adjusting the homogenization time, the number of times of homogenization, or the like. The defibrated extracellular matrix component can also be obtained by carrying out defibration by repeated freezing and thawing.

The fragmented extracellular matrix component may include, at least as a part thereof, an extracellular matrix component obtained by defibration. In addition, the fragmented extracellular matrix component may consist of only the extracellular matrix component obtained by defibration. That is, the fragmented extracellular matrix component may be an extracellular matrix component obtained by defibration. The extracellular matrix component obtained by defibration preferably includes a collagen component (defibrated collagen component) obtained by defibration. The defibrated collagen component preferably maintains the triple-helix structure derived from collagen. The defibrated collagen component may be a component that partially maintains the triple-helix structure derived from collagen.

Examples of the shape of the fragmented extracellular matrix component include a fibrous shape. The fibrous shape means a shape that is composed of thread-shaped collagen components or a shape that is composed of thread-shaped extracellular matrix components that have undergone intermolecular crosslinking. At least a part of the fragmented extracellular matrix component may have a fibrous shape. The fibrous extracellular matrix component includes a thin thread-shaped material (fine fibers) formed by aggregation of a plurality of thread-shaped extracellular matrix molecules, a thread-shaped material formed by further aggregation of fine fibers, materials obtained by defibrating these thread-shaped materials, and the like. The RGD sequence is conserved in the fibrous extracellular matrix component without being disrupted.

The average length of the fragmented extracellular matrix component may be 100 µm or more and 400 µm or less, may be 100 µm or more and 200 µm or less, may be 1 µm or more and 100 µm or less, may be 10 µm or more and 50 µm or less, or may be 20 µm or more and 40 µm or less. In one embodiment, the average length of the fragmented extracellular matrix component may be 5 µm or more and 400 µm or less, may be 10 µm or more and 400 µm or less, may be 22 µm or more and 400 µm or less, or may be 100 µm or more and 400 µm or less. In another embodiment, the average length of the fragmented extracellular matrix component may be 100 µm or less or may be 50 µm or less, and it may be less than 40 µm, may be less than 30 µm, may be less than 15 µm, may be less than 10 µm, or may be less than 1 µm due to the fact that the aggregation of components in the bioink is less likely to occur and the ejection by the 3D printer is smoother. In another embodiment, the average length of the fragmented extracellular matrix component may be 100 nm or more, may be 1 µm or more, may be 10 µm or more, may be 20 µm or more, or may be 30 µm or more. It is preferable that the average length of the majority of the fragmented extracellular matrix components among the total fragmented extracellular matrix components is within the above-described numerical value range. Specifically, it is preferable that the average length of 95% of the fragmented extracellular matrix components among the total fragmented extracellular matrix components is within the above-described numerical value range. The fragmented extracellular matrix component is preferably a fragmented collagen component of which the average length is within the above-described range, and it is more preferably a defibrated collagen component of which the average length is within the above-described range.

The average diameter of the fragmented extracellular matrix component may be 10 nm or more and 30 µm or less, may be 30 nm or more and 30 µm or less, may be 50 nm or more and 30 µm or less, may be 100 nm or more and 30 µm or less, may be 1 µm or more and 30 µm or less, may be 2 µm or more and 30 µm or less, may be 3 µm or more and 30 µm or less, may be 4 µm or more and 30 µm or less, and may be 5 µm or more and 30 µm or less. The fragmented extracellular matrix component is preferably a fragmented collagen component of which the average diameter is within the above-described range, and it is more preferably a defibrated collagen component of which the average diameter is within the above-described range.

The average length and diameter of the fragmented extracellular matrix component can be determined by measuring an individual fragmented extracellular matrix component with an optical microscope and carrying out image analysis. In the present specification, "average length" means the average value of lengths of a measured specimen in the longitudinal direction, and "average diameter" means the average value of lengths of a measured specimen in a direction orthogonal to the longitudinal direction.

The particle size of the fragmented extracellular matrix component may be less than 40 µm. The fragmented extracellular matrix component having a particle size of less than 40 µm is a fragmented extracellular matrix component that passes through a filter having a pore diameter of 40 µm. In a case where the particle size of the fragmented extracellular matrix component is less than 40 µm, the aggregation of components in the bioink is less likely to occur and the ejection by the 3D printer is smoother.

At least a part of the fragmented extracellular matrix component may be crosslinked intermolecularly or intramolecularly. The fragmented extracellular matrix component may be crosslinked within the molecules constituting the fragmented extracellular matrix component or may be crosslinked between the molecules constituting the fragmented extracellular matrix component.

The content of the fragmented extracellular matrix component in the bioink can be appropriately set according to the shape of the structure to be formed, and the like. The content of the fragmented extracellular matrix component may be 1.0 mg/mL or more, 3.0 mg/mL or more, 5.0 mg/mL or more, 10.0 mg/mL or more, 12.0 mg/mL or more, 15.0 mg/mL or more, or 18.0 mg/mL or more, and it may be 45.0 mg/mL or less, 40.0 mg/mL or less, 35.0 mg/mL or less, 30.0 mg/mL or less, or 25.0 mg/mL or less based on the total volume of the bioink, due to the fact that the strength of the structure formed by bioprinting is further improved. The content of the fragmented extracellular matrix component may be 5 mg/mL or more and 45 mg/mL or less, 5 mg/mL or more and 40 mg/mL or less, 5 mg/mL or more and 35 mg/mL or less, 5 mg/mL or more and 30 mg/mL or less, 5 mg/mL or more and 25 mg/mL or less, 10 mg/mL or more and 45 mg/mL or less, 10 mg/mL or more and 40 mg/mL or less, 10 mg/mL or more and 35 mg/mL or less, 10 mg/mL or more and 30 mg/mL or less, 10 mg/mL or more and 25 mg/mL or less, 15 mg/mL or more and 45 mg/mL or less, 15 mg/mL or more and 40 mg/mL or less, 15 mg/mL or more and 35 mg/mL or less, 15 mg/mL or more and 30 mg/mL or less, or 15 mg/mL or more and 25 mg/mL or less based on the total volume of the bioink, due to the fact that the strength of the structure formed by bioprinting is further improved.

The bioink may further contain a structure forming material. The structure forming material is a material that can form a structure by bioprinting. The structure forming material does not contain a component that corresponds to the above-described fragmented extracellular matrix component. The structure forming material may be dissolved or dispersed in the bioink. A commercially available bioink material can be used as the structure forming material. The kind of the structure forming material can be appropriately selected according to the shape of the structure, the use application, and the like. The structure forming material may be, for example, an extracellular matrix component. As the extracellular matrix component, those exemplified as above may be used.

Specifically, examples of the structure forming material include collagen, fibrin, chitosan, nanocellulose, polylactic acid (PLA), polycaprolactone (PCL), hydroxyapatite (HA), tricalcium β-phosphate (β-TCP), alginic acid, and gelatin methacryloyl.

The content of the structure forming material can be appropriately set according to the shape of the structure, the use application, and the like. The content of the structure forming material may be, for example, 0.1% by mass or more, 0.2% by mass or more, or 0.5% by mass or more, and it may be 10% by mass or less, 5% by mass or less, or 2% by mass or less based on the total mass of the bioink.

The bioink may further contain an aqueous medium. Examples of the aqueous medium include saline such as phosphate buffered saline (PBS), sterile water, a pH buffer solution such as a Good's buffer.

The bioink may contain cells or may not contain cells. The bioink may further contain components (other components) other than the components described above.

Various conditions for the pH, viscosity, and the like of the bioink can be appropriately set according to the composition of the bioink, the use application of the structure, the printer to be used, and the like. The pH of the bioink may be, for example, 5 to 8.0, 6.0 to 8.0, or 6.5 to 7.5.

The bioink according to the present embodiment can be obtained, for example, by a method including a step of mixing a fragmented extracellular matrix component with a structure forming material in an aqueous medium.

### [Structure]

The structure according to the present embodiment is a printed matter of the above-described bioink, which can be obtained by carrying out printing with the above-described bioink.

The structure according to the present embodiment may be a structure that does not contain cells or may be a structure that contains cells (a cell structure). In the present specification, the term "cell structure" means an aggregate of cells (a clumpy cell population) in which cells are three-dimensionally disposed through extracellular matrix components, where the aggregate is an aggregate that is artificially made by cell culture. The cell structure can be obtained by carrying out printing with a bioink containing cells.

The cell is not particularly limited; however, it may be a cell derived from a mammalian animal such as a human, a monkey, a dog, a cat, a rabbit, a pig, a cow, a mouse, or a rat. The site from which a cell is derived is also not limited. The cell may be a somatic cell derived from bone, muscle, an internal organ, nerve, brain, bone, skin, blood, or the like, or it may be a reproductive cell. Further, the cell may be a stem cell and may be a cultured cell such as a primary cultured cell, a subcultured cell, or a cell line cell.

The shape of the structure is not particularly limited, and examples thereof include a sheet shape, a fiber shape, a spherical shape, a substantially spherical shape, an ellipsoidal shape, a substantially ellipsoidal shape, a hemispherical shape, a substantially hemispherical shape, a semicircular shape, a substantially semicircular shape, a rectangular shape, and a substantially rectangular parallelepiped shape. The structure may be adhered to a support or may not adhered to a support.

The structure according to the present embodiment can be manufactured by a method including carrying out printing with the bioink described above and solidifying the bioink used for the printing. In the method, the solidification may be allowed to proceed while carrying out printing with the bioink. Alternatively, printing may be carried out with the bioink to form a precursor of a structure having a desired shape, and then the precursor of the structure may be solidified to form the structure.

The method of carrying out printing with the bioink can be appropriately selected according to the use application of the structure, the composition of the bioink, and the like. Specific examples of the method of carrying out printing with a bioink include an inkjet method, a material extrusion method, a laser transfer method, and an optical shaping method. In addition, the printing may be carried out manually with a syringe or the like without using a specific printer device.

The bioprinter may be any commercially available type such as INKREDIBLE (trademark), INKREDIBLE+ (trademark), or BIO X (trademark), as a 3D printer of CELLINKAB, or any robot type bioprinter in the related art, which is equipped with standard constitutional elements such as a motor, a printhead, a printed board, a print structure, a cartridge, a syringe, a platform, a laser, and a control device.

The conditions for printing (for example, temperature, print pressure, and nozzle) can be appropriately set according to the shapes and use applications of the printer and the structure. The temperature at the time of printing may be, for example, 4°C or more or may be 40°C or less. The print pressure may be, for example, 1 kPa or more or may be 200 kPa or less.

The method of solidifying the bioink used for the printing can be appropriately selected according to the composition of the bioink, and the like. As the method of solidifying the bioink used for the printing, a method of carrying out solidification by stimulation such as light or heat, elapse of time, contact with a liquid medium or the like can be used.

The bioink used for the printing may be solidified in a liquid medium by ejecting it from a printer or the like to the liquid medium. The liquid medium for solidification can be appropriately set according to the composition of the bioink, and the like. The pH of the liquid medium may be, for example, 5 to 8.

One example of the liquid medium for solidification is a liquid medium in which particles are dispersed (hereinafter referred to as a "particle dispersion medium"). In a case where the liquid medium for solidification is a particle dispersion medium, the shape of the ejected bioink or the precursor of the structure formed by the ejection is retained by the particles in the liquid medium, whereby it is much easier to form a structure having high strength.

Particles in the particle dispersion medium may contain biogum. The biogum means a polysaccharide that is produced by a microorganism, a living body such as a plant. Examples of the biogum include a microbial biogum or a vegetable biogum. Examples of the microbial biogum include gellan gum, xanthan gum, diutan gum, Welan gum, and Pullulan gum. Examples of the vegetable biogum include acacia gum, tara gum, glucomannan, pectin, locust bean gum, guar gum, carrageenan, and tragacanth. The biogum may be a gellan gum since it is particularly suitable for forming a structure having high strength.

The particle diameter in the particle dispersion medium may be, for example, 10 µm or more, 20 µm or more, 30 µm or more, or 40 µm or more, and it may be 100 µm or less, 80 µm or less, or 60 µm or less. In a case where the particle diameter is within the above-described range, it is much easier to form a structure having high strength. The particle diameter can be measured with an image obtained by using a confocal laser scanning microscope (for example, FV3000). Specifically, the particle diameter can be measured by acquiring an image of the particle dispersion medium by using a confocal laser scanning microscope, carrying out image analysis by ImageJ, and manually calculating the particle diameter. More detailed test conditions are as described in Examples below.

The particle diameter of the particles in the particle dispersion medium can be controlled by adding citric acid to the particle dispersion medium and adjusting the adding amount. In a case where the particle dispersion medium contains citric acid, the content of citric acid may be, based on the total amount of particle dispersion medium, more than 0 mol/L, 0.10 mol/L or more, 0.20 mol/L or more, or 0.25 mol/L or more, and it may be 1.0 mol/L or less, 0.80 mol/L or less, 0.60 mol/L or less, or 0.40 mol/L or less.

The particle dispersion medium containing biogum can be obtained, for example, by the following method. The biogum is dissolved in a liquid medium (for example, phosphate buffered saline) to obtain a biogum solution. The concentration of biogum can be appropriately set according to the kind of biogum and the like. In a case where gellan gum is used as the biogum, the concentration of the biogum may be, for example, 0.3% to 0.7% by mass with respect to the total amount of the biogum solution. A predetermined treatment required for gelation, for example, allowing the biogum solution to stand for a predetermined period of time, is carried out for each substance, whereby gelation is carried out to obtain a biogum gel. The biogum gel is formed into particles by homogenization to obtain a particle solution of the biogum. A citric acid buffer solution is added to the particle solution of the biogum, homogenization is further carried out, and as necessary, centrifugation for degassing is carried out, whereby a particle dispersion medium can be obtained.

Another example of the liquid medium for solidification is a liquid containing an organic solvent. In a case where the bioink contains collagen as a structure forming material, for example, a mixture of acetonitrile and water can also be used as a liquid medium for solidification.

In the structure according to the present embodiment, the diameter, the length, and/or the width may be, for example, 0.1 mm or more and may be 50 cm or less.

The Young's modulus of the structure according to the present embodiment may be 0.1 MPa or more, 0.2 MPa or more, or 0.3 MPa or more, and it may be 1.0 MPa or less, 0.6 MPa or less, or 0.4 MPa or less.

The Young's modulus of the structure can be measured using a small tabletop tester EZ-test (manufactured by Shimadzu Corporation). Regarding a sheet-shaped structure, a sheet-shaped structure having a size of 30 mm × 6 mm × 0.6 mm is prepared, and the structure is subjected to a tensile test with a load cell of the tester under conditions of a temperature of 25°C, a tensile speed of 10.0 mm/min, and an initial distance between clamps of 15 mm, whereby a stress-strain line (Stress-Strain curve) is obtained. The Young's modulus (MPa) can be calculated from the slope of the elastic deformation region at the initial stress rise in the obtained stress-strain line.

The structure according to the present embodiment can be suitably used, for example, as a scaffold material for cell culture or tissue formation, as a replacement for an experimental animal, or as a transplant material.

### Examples

Hereinafter, the present invention will be more specifically described based on test examples. However, the present invention is not limited to test examples below.

### Experimental Example 1: Production of gellan gum (GG) bath

### <Material>

· GG powder: Gellan gum powder (trade name: KELCOGEL AFT; manufactured by Sansho Co., Ltd.)

### <Procedure>

The GG powder was dissolved in PBS at 0.5 ppm by mass to obtain a GG solution. The GG solution was subjected to gelation under conditions of 6 h and room temperature to obtain a GG gel. Using a homogenizer, the GG gel was homogenized and formed into particles under conditions of a power grade of 6 and a time of 6 minutes. 4.2 mL of a citric acid buffer solution (concentration: 0.5 M, 1 M, 1.5 M, or 2 M) was added to the 10 mL of a GG particle solution obtained by the above procedure. In addition, the GG particle solution to which the citric acid buffer solution had been added was further homogenized and further formed into particles under the conditions of the same homogenizer, the same power (grade: 6), and 2 minutes as those in the production of CMF described later. Thereafter, degassing by centrifugation was carried out to produce a GG-TSC particle bath (particle dispersion medium).

### Experimental Example 2: Observation of particle diameter in GG-TSC particle bath

### <Observation method>

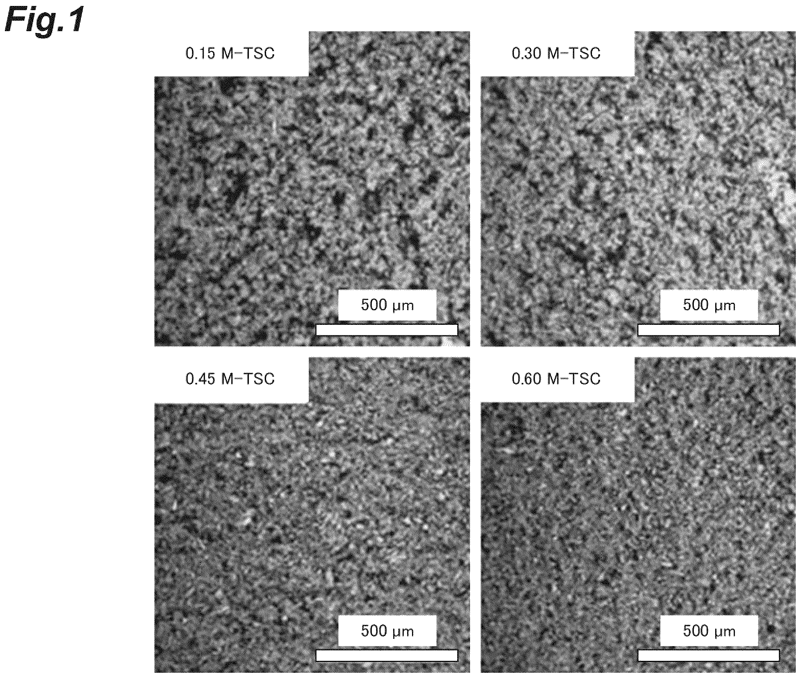

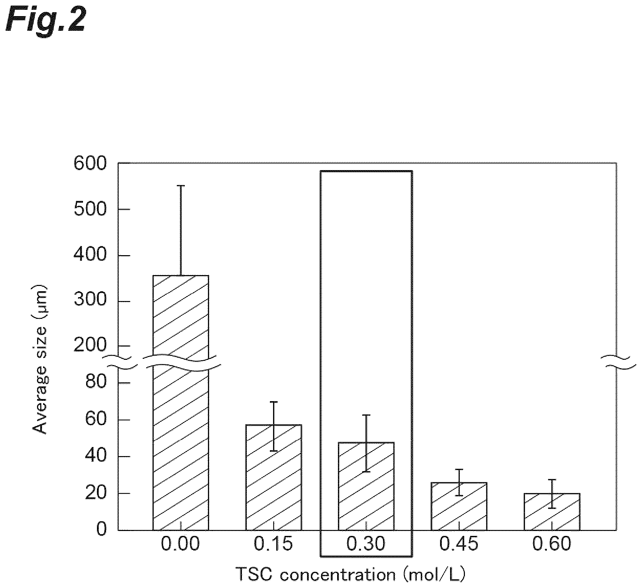

The GG-TSC particle bath was observed with a confocal laser scanning microscope (FF3000), and the particle diameter was manually calculated by image analysis with ImageJ (n = 50 per one condition). FIG. 1 and FIG. 2 show results obtained by observing particles and results obtained by measuring particle diameters.

### <Result>

As shown in FIG. 1 and FIG. 2, the particle diameter in the GG-TSC particle bath was reduced to a range of 20 to 50 µm by the addition of citric acid.

### Experimental Example 3: Preparation of bioink and printing of fiber-shaped structure

### <Material>

· Type I Collagen Powder: (Nippi, Inc., ASC-1-100)

### <Procedure>

### Preparation of collagen solution

A type I collagen powder was suspended in 10× PBS so that the collagen content was 1% by mass, and then homogenized with the same homogenizer, at the same power (grade: 6), and at the same time (6 min) as those in the production of CMF. Next, centrifugation was carried out under conditions of 10,000 rpm and 10 min, and the supernatant was removed. Thereafter, 1 × PBS was added thereto and then allowed to stand one day at 4°C to obtain a collagen solution.

### Preparation of bioink containing fragmented collagen component (CMF)

50 mg of a type I collagen powder was suspended in 5 mL of PBS, homogenized at room temperature using a homogenizer for 6 minutes, and then centrifuged under conditions of 10,000 rpm, 10 minutes, and room temperature to obtain a CMF solution containing the fragmented collagen component (CMF) as a precipitate. The supernatant was removed from the solution, and 2 mL of the above-described collagen solution of 1% by mass was added thereto. Thereafter, homogenization was carried out for 1 minute, and air bubbles were removed by vacuuming, thereby preparing a bioink containing collagen and the fragmented collagen component. The final concentration of CMF in the bioink was 40 mg/mL.

The average length of the fragmented collagen component (the average value of the length of the measured specimen in the longitudinal direction) was 35 µm.

### <Production of fiber-shaped (thread-shaped) structure>

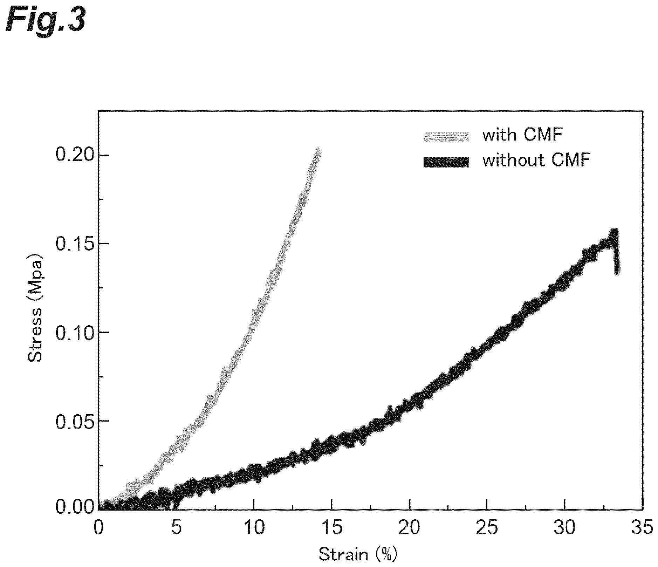

The bioink was ejected (formed into a thread shape) in a solvent of 60:40 = acetonitrile (ACN):water with a syringe equipped with a 20 G needle. Then, the solvent was replaced with 50% EtOH, and the ejected bioink was allowed to stand for 1 hour. Thereafter, the solvent was replaced with 50% ethanol containing 0.2% by mass of glutaraldehyde (GA), and the ejected bioink was allowed to stand for 12 hours. The thread-shaped structure obtained from the above procedure was washed with PBS and subjected to a test for tensile strength with the EZ-test. The results are shown in FIG. 3. The test conditions for the test (for fiber structure), the results of which are shown in FIG. 3, were set as follows.
· Fiber diameter: 600 µm
· Tensile speed: 10.0 mm/min
· Initial distance between clamps: 30 mm

From FIG. 3, the structure of the example which was produced using a bioink containing CMF exhibited a remarkably high tensile strength as compared with the structure of the comparative example which was produced using a bioink containing no CMF. As a result of calculating the Young's modulus (MPa) from the slope at the initial stress rise in the stress-strain line, the Young's modulus (Modulus) of the structure of the example which was produced using a bioink containing CMF was 1 to 2 MPa. On the other hand, the Young's modulus of the structure of the comparative example which was produced using a bioink containing no CMF was 0.3 to 0.8 MPa. It was shown that the strength of the structure is improved in a case where a bioink containing CMF is used.

### Experimental Example 4: Filtration and observation of CMF (in PBS)

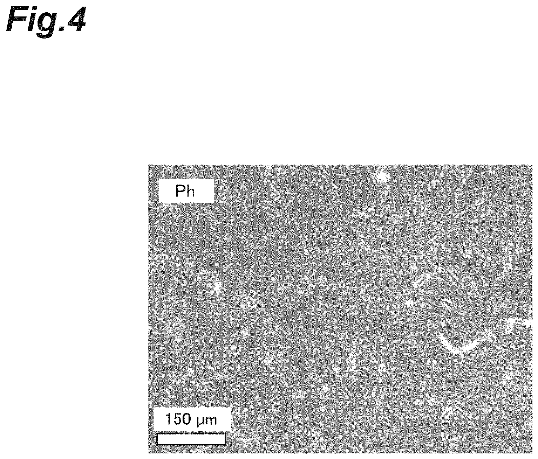

In the same manner as in Experimental Example 3, 50 mg of a type I collagen powder was suspended in 5 mL of PBS and homogenized for 6 minutes at room temperature using a homogenizer to prepare a PBS suspension of CMF. The PBS suspension of CMF was filtered with a cell strainer of 40 µm and subjected to a microscopic observation in PBS. FIG. 4 shows the results of the microscopic observation. The yield of the filtered CMF with respect to the mass after lyophilization was 85%.

### Experimental Example 5: Observation of filtered CMF (in bioink)

The filtered CMF prepared in Experimental Example 4 was added to a 1% collagen solution to prepare a bioink (the procedure for preparing the bioink was the same as in Experimental Example 3, except that the filtered CMF was used). Thereafter, the obtained bioink was subjected to a microscopic observation.

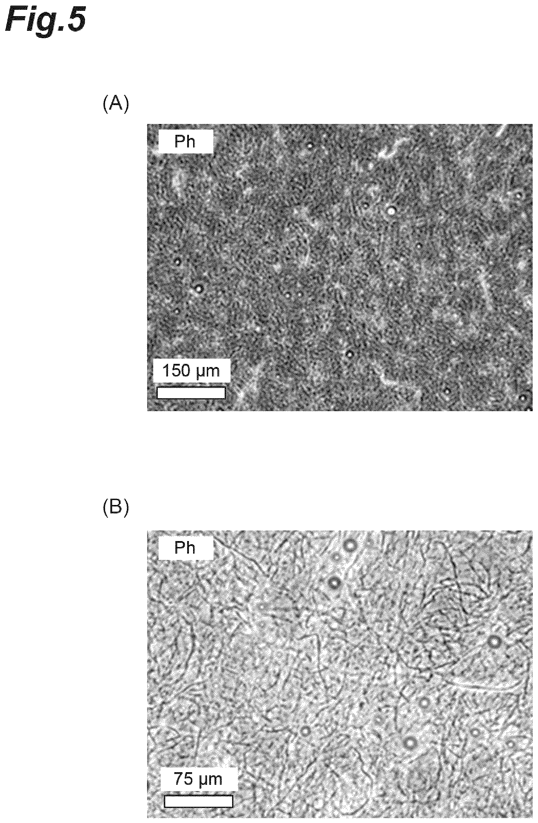

As shown in FIG. 5, in the bioink, CMF was well dispersed in the collagen solution. No large aggregates of CMF were found in the bioink. This indicates that the obtained bioink can be used more suitably for printing.

CMF's having micrometer-scale lengths are intertwined with each other, which presumably contributes to the improvement of the mechanical strength.

### Experimental Example 6: Printing with bioink containing filtered CMF

A sheet-shaped structure (30 mm × 6 mm × 0.6 mm) was designed. A CMF-free bioink or the CMF-containing bioink of Experimental Example 5 (a CMF concentration of 10 or 20 mg/mL) was ejected into a GG-0.3 MTSC particle bath to print the designed sheet-shaped structure. Thereafter, the printed sheet-shaped structure was allowed to stand for 1 hour. Detailed printing conditions were set as follows.

### (Printing conditions)

Bath: GG-0.3 MTSC particle bath;
Ink: CMF-containing bioink;
CMF concentration: 10 mg/mL or 20 mg/mL in 1% by mass collagen solution;
Nozzle: 25 G (250 µm);
Air-driven pressure: 20 kPa;
Moving speed: 25 mm/s;
Layer number: 6 layers;
Layer height: 0.1 mm;
Infill: 100%;
Infill texture: rectilinear 45°;
Printing device: BIO X (Cellink Corporation)

The CMF-containing bioink in PBS could be continuously extruded without clogging the needle. The printed structure had a clear contour and was approximated to the designed model.

The sheet-shaped structure obtained by carrying out printing with the bioink (CMF-containing or CMF-free bioink) was fixed with glutaraldehyde in the same manner as in the tensile test using the fiber-shaped structure. Specifically, a liquid medium in the obtained sheet-shaped structure was replaced with 50% EtOH. Thereafter, 50% EtOH was replaced with 50% ethanol containing 0.2% by mass glutaraldehyde (GA), and the sheet-shaped structure was allowed to stand for 12 hours at 37° C. Thereafter, the sheet-shaped structure was subjected to two times of solvent replacement by immersion in 20 mL PBS for 3 hours. As a result, a sample for testing was obtained.

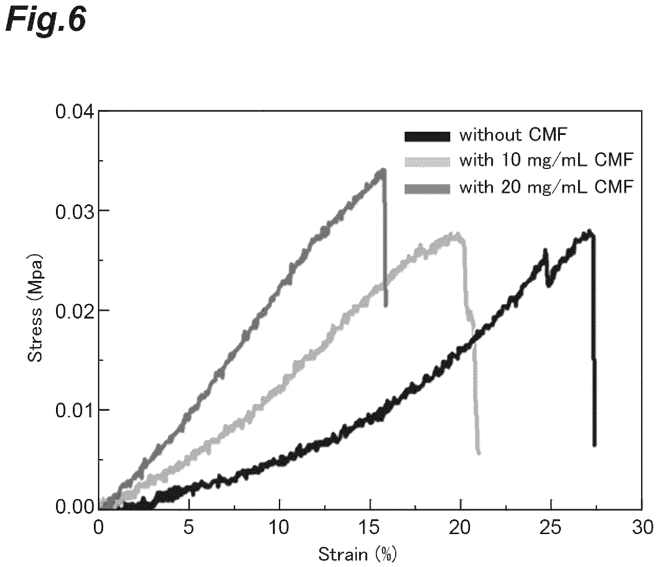

The sample for testing was subjected to a tensile test using the Ez-test. FIG. 6 shows the result of the tensile test. The test conditions for the test (for sheet structure), the results of which are shown in FIG. 6, were set as follows.
· Sheet diameter: 30mm × 6mm × 0.6mm
· Tensile speed: 10.0 mm/min
· Initial distance between clamps: 15 mm

The structure which was produced using a bioink containing CMF had improved tensile strength as compared with the structure which was produced using a bioink containing no CMF. The structure produced using a bioink having a CMF content of 20 mg/mL was confirmed to have improved tensile strength as compared with the structure which was produced using a bioink containing no CMF.

### Experimental Example 7: Optimization of concentration of CMF/collagen solution

The production of a bioink and a sheet-shaped structure in which the collagen content (excluding the CMF content) is 1%, 2%, or 3% by mass based on the total mass of the bioink, and the CMF content is 0 mg/mL, 10 mg/L, or 20 mg/mL, and the production of a sample for testing was carried out in the same manner in Experimental Example 5 to prepare the bioink, the sheet-shaped structure, and the sample for testing.

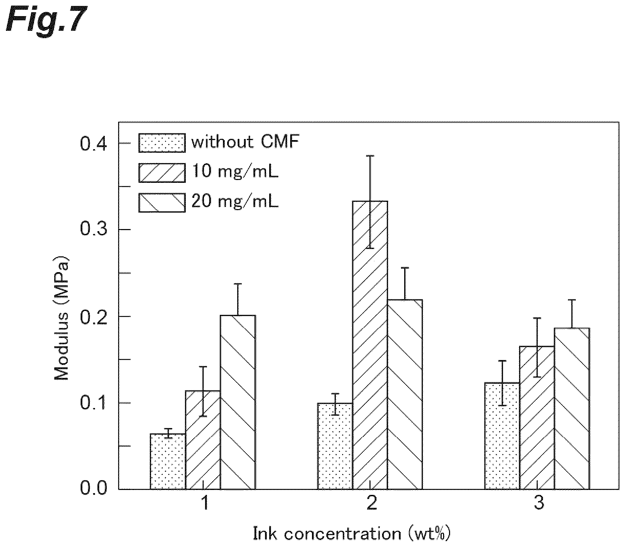

The ample for testing was subjected to a tensile test in the same manner as in Experimental Example 3 and Experimental Example 6. FIG. 7 shows the results obtained by calculating the Young's modulus (MPa) from the slope at the initial stress rise in the stress-strain line. The test conditions for the test (for sheet structure), the results of which are shown in FIG. 7, were set as follows.
· Sheet diameter: 30mm × 6mm × 0.6mm
· Tensile speed: 10.0 mm/min
· Initial distance between clamps: 15 mm

A high Young's modulus tended to be obtained by increasing the ink concentration (the collagen content in the bioink). The improvement of Young's modulus by CMF was most remarkable in a case where the ink concentration was 2% by mass. The conditions in which the ink concentration was 2% by mass and the ink contained 10 mg/ml CMF gave particularly excellent results.

## Claims

1. A bioink comprising:
a fragmented extracellular matrix component.

2. The bioink according to claim 1, wherein the fragmented extracellular matrix component comprises a fragmented collagen component.

3. The bioink according to claim 1 or 2, further comprising:
a structure forming material.

4. The bioink according to claim 3, wherein the structure forming material comprises an extracellular matrix component.

5. The bioink according to claim 4, wherein the extracellular matrix component comprises collagen.

6. The bioink according to claim 1 or 2, wherein a content of the fragmented extracellular matrix component is 5 ng/mL or more and 30 ng/mL or less based on a total amount of the bioink.

7. The bioink according to claim 1 or 2, wherein the fragmented extracellular matrix component has a particle size of less than 40 µm.

8. A method for producing a structure, comprising:
carrying out printing with the bioink according to claim 1 or 2 and; and
solidifying the bioink used for the printing.
